# EUROPEAN PATENT APPLICATION

(11) **EP 4 159 861 A1**
(43) Date of publication of application: **05.04.2023**
(21) Application number: 21814439.2
(22) Date of filing: 25.05.2021
(51) Int. Cl.: C12N 15/52, C12N 15/63, C12N 9/00

(54) **METHOD FOR PREPARING PLASMID CONTAINING TYPE I POLYKETIDE SYNTHASE GENE**

(30) Priority: 26.05.2020 JP 2020091798
(71) Applicant: Spiber Inc., Tsuruoka-shi, Yamagata 997-0052 (JP)
(72) Inventor: LIPS David, Tsuruoka-shi, Yamagata 997-0052 (JP); VERKLEIJ Gijs, Tsuruoka-shi, Yamagata 997-0052 (JP)
(74) Representative: Handley, Matthew Edward
(86) International application number: PCT/JP2021/019819
(87) International publication number: WO 2021/241584

(57) **Abstract**

The present invention relates to a method for preparing a plasmid containing DNA that encodes a Type I polyketide synthase (PKS), the method including a step of introducing a DNA construct containing tandem repeats of DNA that encodes a PKS into a Bacillus subtilis competent cell.

## Description

### Technical Field

The present invention relates to a method for preparing a plasmid containing a Type I polyketide synthase (PKS) gene.

### Background Art

A natural compound produced from microorganisms such as actinomycetes and filamentous fungi is known as a useful substance having a wide variety of structures and biological activities. At present, it is easy to specify a gene cluster for biosynthesis of a useful substance by decoding a genome. In addition, it has also become clear that there are a large number of gene clusters that biosynthesize useful substances unused by humans. Among secondary metabolites of microorganisms, a gene cluster for biosynthesis of industrially important polyketide-based compounds and peptide-based compounds has been mainly studied. Examples thereof include a Type I polyketide synthase (PKS) used for biosynthesis of macrolide compounds such as erythromycin, FK-506 (tacrolimus), rapamycin, and avermectin which are clinically applied among secondary metabolites produced by actinomycetes.

In consideration of the difficulty of producing a polyketide compound by a traditional chemical method and the normally low production of polyketides in wild-type cells, there is considerable interest in finding improved or alternative means for producing a polyketide compound. For these reasons, heterologous production of a compound has been attempted by introducing a gene cluster required for biosynthesis into other cells from the original strain. In actual fact, the conventional methods of heterologous expression are often limited to a small gene cluster (< 40 kb), and many PKS gene clusters are much larger than this, ranging from a few kilobases to 100 kilobases or more of DNA.

As a method of assembling DNA, a method of linking a plurality of DNA fragments, such as the Golden Gate method or the Gibson method, is known (Non Patent Literature 1).

The Golden Gate method is a method in which a plurality of DNA fragments having a base sequence recognized by a Type IIs restriction enzyme are prepared at one or both ends, and the plurality of DNA fragments is treated with a Type IIs restriction enzyme and DNA ligase. The plurality of DNA fragments is hybridized by an adhesive end (sticky-end) generated by cleavage by the Type IIs restriction enzyme, and then nicks are joined by the DNA ligase, such that a DNA fragment having a desired base sequence can be produced. By designing the type and arrangement of the base sequence recognized by the Type IIs restriction enzyme, the plurality of DNA fragments cleaved by the Type IIs restriction enzyme are linked so as to eliminate a recognition site of the restriction enzyme, such that a DNA fragment having a desired base sequence can be produced. A method of introducing a DNA fragment having a desired base sequence into a cloning vector using the Golden Gate method has been reported (Patent Literature 1 and Non Patent Literature 1).

The Gibson method is a method in which a plurality of DNA fragments designed so that linking regions of the respective end portions of DNA fragments linked adjacent to each other overlap (have the same base sequence) by about 15 to 80 base pairs (bp) are prepared, and the plurality of DNA fragments is treated with a 5' exonuclease, a DNA polymerase, and DNA ligase. A single-stranded DNA is partially produced from the end of the DNA fragment by the 5'exonuclease. The resulting single-stranded DNA hybridizes at an overlapping base sequence part. Thereafter, a gap is filled with a DNA polymerase, and nicks are joined by DNA ligase, such that a DNA fragment having a desired base sequence can be produced.

In the Gibson method, since it is not necessary to have a base sequence recognized by a restriction enzyme, there is no restriction on the base sequence, and the Gibson method is also suitable for constructing a long DNA fragment (Non Patent Literature 2 and Patent Literatures 2 and 3).

### Citation List

### Patent Literatures

Patent Literature 1: US 2010/0291633 A
Patent Literature 2: US 7,723,077
Patent Literature 3: JP 2011-512140 A

### Non Patent Literatures

Non Patent Literature 1: PLoS One, 2009, 4(5), e5553
Non Patent Literature 2: Chemistry and Biology, 2016, Vol.54, No.10, pp.740 to 746

### Summary of Invention

### Technical Problem

In the Golden Gate method, the number of DNA fragments that can be linked at a time is about 20, and it is said that when the number exceeds this, the efficiency is reduced. Since a sequence such as a PKS gene cluster is a repeat sequence or a base sequence having a high guanine-cytosine (GC) content, the frequency of mis-ligation tends to be high, and therefore it is difficult to accurately link a plurality of DNA fragments at a time.

Even in the Gibson method, when assembling a PKS gene cluster having a repeat sequence or a base sequence having a high GC content, it is considered that the accuracy of hybridization of a single-stranded DNA is reduced, and thus the frequency of mis-ligation is extremely high. In practice, an assembly scale is limited to a maximum of 15 fragments.

An object of the present invention is to efficiently synthesize a polyketide synthase gene.

### Solution to Problem

The present invention provides a plasmid containing the following Type I polyketide synthase gene, a method for preparing the plasmid, and a method for producing a PKS enzyme.
[1] A method for preparing a plasmid containing DNA that encodes a Type I polyketide synthase (PKS), the method including a step of introducing a DNA construct containing tandem repeats of DNA that encodes a PKS into a Bacillus subtilis competent cell.
[2] The method according to [1], further including:
   a step of preparing a plurality of DNA fragments having sticky ends at both ends by cleaving a plurality of DNA fragment precursors with a Type II restriction enzyme; and
   a step of preparing the DNA construct by linking the plurality of DNA fragments.
[3] The method according to [2], further including a step of preparing tandem repeats of DNA that encodes a Type I polyketide synthase (PKS) by mixing solutions each containing the plurality of DNA fragments at a molar concentration ratio of the DNA fragments in each solution of 0.8 to 1.2.
[4] The method according to [2] or [3], in which a guanine-cytosine (GC) content in the DNA fragment precursor is 65% or less.
[5] The method according to any one of [1] to [4], in which the step of introducing the DNA construct is a step of co-culturing the DNA construct and the Bacillus subtilis competent cell.
[6] The method according to any one of [1] to [5], further including a step of recovering plasmid DNA from Bacillus subtilis into which the DNA construct is introduced.
[7] A plasmid containing DNA that encodes a Type I polyketide synthase (PKS), in which the plasmid is obtained by the method according to any one of [1] to [6].
[8] A method for producing a Type I polyketide synthase (PKS), the method including: a step of transforming a host cell with a plasmid; and a step of culturing the transformed host cell, in which the plasmid is the plasmid containing DNA that encodes a PKS obtained by the method according to any one of [1] to [6].
[9] The method according to [8], in which the host cell is a bacterium of the genus Streptomyces.

### Advantageous Effects of Invention

It is possible to provide a method for accurately and efficiently assembling a long chain DNA that encodes a large gene cluster such as a PKS.

### Brief Description of Drawings

Fig. 1 is a schematic view illustrating a target sequence in which three 6-deoxyerythronolide B synthase (DEBS) Type I polyketide synthase (PKS) gene-encoding sequences are linked, a promoter and a ribosome binding sequence (RBS) are integrated before each of the sequences, and a terminator sequence is integrated after each of the sequences.
Fig. 2 illustrates results of electrophoresis of restriction enzyme decomposition products of a PKS gene contained in a plasmid obtained in Comparative Example 1 using E. coli.
Fig. 3 illustrates results of electrophoresis of restriction enzyme decomposition products of a PKS gene contained in a plasmid obtained in Example 1 using Bacillus subtilis, in which the checks in Fig. 3 indicate that a desired DEBS PKS gene-encoding sequence is obtained.

### Description of Embodiments

Hereinafter, embodiments of the present invention will be described in detail. However, the present invention is not limited to the following embodiments.

A method for preparing a plasmid containing DNA that encodes a Type I polyketide synthase (hereinafter, may be simply described as a "PKS" or as a "Type I PKS") according to the present embodiment includes a step of introducing a DNA construct containing tandem repeats of DNA that encodes a PKS into a Bacillus subtilis competent cell.

Herein, a DNA construct containing tandem repeats of DNA that encodes a PKS (hereinafter, may be referred to as a "PKS gene") can be converted into a plasmid containing one or a plurality of PKS genes in a cell of Bacillus subtilis. For example, the DNA construct is introduced into a cell of Bacillus subtilis by co-culturing the DNA construct and the Bacillus subtilis competent cell. Next, the DNA construct is circularized utilizing the homology of repeat units containing DNA that encodes a PKS within the cell of Bacillus subtilis to be converted into a plasmid containing DNA that encodes one or a plurality of PKSs. Therefore, a plasmid containing DNA that encodes a PKS can be obtained from Bacillus subtilis.

As a method for making Bacillus subtilis competent cells, the method described in Anagnostopoulou, C. and Spizizen, J., J. Bacteriol., 81, 741-746 (1961) is preferably used.

The amount of DNA construct solution added to the Bacillus subtilis competent cell is not particularly limited. The amount of the DNA construct solution is preferably 1/20 to the same amount, and more preferably a half amount, with respect to the amount of a culture solution of the Bacillus subtilis competent cell.

At least one DNA that encodes a PKS, preferably one DNA that encodes a PKS, is integrated into the plasmid when the plasmid is formed, such that the tandem repeats of DNA that encodes a PKS are obtained. As a method for purifying a plasmid from Bacillus subtilis, a known method can be used.

It can be confirmed by a size pattern of a fragment generated by a restriction enzyme cleavage, a PCR method, and a base sequencing method that the plasmid obtained by the method described above contains desired DNA that encodes a PKS.

The DNA construct may contain DNA that encodes a Type I polyketide synthase (PKS) (for example, DNA that encodes a domain included in a PKS), and the type of PKS is not particularly limited. In addition, a size of the DNA construct is not particularly limited. The type of DNA that encodes a PKS (for example, DNA that encodes a domain included in a PKS) is not particularly limited, and may be DNA having a naturally derived sequence such as a microorganism or DNA having an artificially designed sequence. In the naturally derived sequence, one codon is mainly used by the organism species to express the corresponding amino acid, but in the case of heterologous expression, it is preferable to use an artificially designed sequence adapted to the codon usage frequency of the host. Examples of other factors that may affect the results of heterologous expression include a guanine-cytosine (GC) content (a total content of guanine and cytosine in the base sequence) and a repeat sequence. The repeat sequence reduces genetic stability, causes a risk of incorrect hybridization, and inhibits the synthesis of repeat segments. Therefore, in the case of heterologous expression, the DNA that encodes the PKS is preferably optimized in relation to codon usage and GC content. However, these requirements are usually difficult to be satisfied optimally at the same time. For example, the optimization of the codon can lead to highly repetitive DNA sequences or high GC content.

In the present invention, the GC content in the DNA that encodes the PKS is 30 to 70%. The GC content in the DNA that encodes the PKS is preferably 70% or less, 68% or less, 65% or less, or 60% or less. By using the method according to the present invention, it is possible to synthesize a target plasmid having high efficiency even when the GC content in the DNA that encodes the PKS is 50% or more, 52% or more, 55% or more, 58% or more, or 60% or more. In the present invention, the DNA that encodes the PKS optimizes a codon so that repetition of base sequences of 20 bp or more does not appear. It is preferable to avoid an extreme difference in GC content in the DNA that encodes the PKS. For example, the difference in GC content between the highest and lowest 50 bp stretches is preferably 52% or less. It is preferable to minimize homopolymers. It is preferable to minimize the number/length of small repeat sequences dispersed within the DNA that encodes the PKS as much as possible. The length of the repeat sequence is short, and the total number of 5 to 10 bp repeats is preferably 150 or less, 120 or less, 100 or less, 80 or less, or 60 or less. By using the present invention, the number of 5 to 10 bp long repeats contained in the gene that encodes a PKS may be 40 or more, 45 or more, or 50 or more.

The plasmid containing the PKS gene obtained in the present invention can produce polyketide with high efficiency in combination with a specific host. In one preferred embodiment, the present invention can utilize genetically manipulated host cells in which the naturally occurring PKS gene is substantially deleted. These host cells can be transformed with plasmids containing various PKS genes for production of active polyketides. The present invention provides production of a large amount of product at an appropriate stage of the growth cycle. The polyketides thus produced can be used to treat many diseases as a therapeutic agent depending on the type of polyketide. For example, it has been found that some polyketides produced by a host transformed with the plasmid of the present invention are used as immunosuppressive agents or antitumor agents in order to treat viral, bacterial and parasitic infections. The ability to recombinantly produce polyketides also provides a powerful tool to characterize the PKS and the mechanism of action thereof.

More preferably, the host cells for recombinant production of the target polyketide may be derived from any organism that can be transformed with the plasmid of the present invention. Therefore, the host cells of the present invention may be derived from either prokaryotes or eukaryotes. However, a preferred host cell is one constructed from an actinomycete, and more preferably a host of the genus Streptomyces. The greatest merit of using the host of the genus Streptomyces is that a production titer is higher than that in heterologous expression production using E. coli, and there is a post-translational modification system that is essential for the activity expression of the Type I PKS. Specific examples thereof include S. albus, S. ambofaciens, S. avermitilis, S. azureus, S. cinnamonensis, S. coelicolor, S. curacoi, S. erythraeus, S. fradiae, S. galilaeus, S. glaucescens, S. hygroscopicus, S. lividans, S. parvulus, S. peucetius, S. rimosus, S. roseofulvus, S. thermotolerans, and S. violaceoruber, and S. albus is preferable.

The host cells can be genetically manipulated using standard techniques (for example, homologous recombination) by deleting the naturally occurring PKS gene from the host cells.

The DNA that encodes the PKS may be naturally occurring, modified in codon usage, or modified in one or two or more amino acids. In one preferred embodiment, a Streptomyces PKS contains the products of three open reading frames (ORF1, ORF2, and ORF3). The PKS includes three domains: a keto synthase (KS) domain, an acyl transferase (AT) domain, and an acyl carrier protein (ACP) domain, and these three domains can extend a polyketide chain. The PKS may further include a domain involved in modification of a main chain, such as a keto reductase (KR) domain, a dehydrase (DH) domain, or an enoyl reductase (ER) domain. Examples of a compound prepared by the PKS include 6-deoxyerythronolide B (6-dEB), frenolicin, granaticin, tetracenomycin, 6-methylsalicylic acid, oxytetracycline, tetracycline, erythromycin, griseusin, nanomycin, medermycin, daunorubicin, tyrosine, carbomycin, spiramycin, avermectin, monensin, nonactin, curamycin, rifamycin, lipomycin, and candicidin.

The plasmid has a control sequence operably linked to desired DNA that encodes a PKS. An expression system suitable for use in the present invention includes a system that functions in a eukaryotic host cell and a prokaryotic host cell. However, as described above, a prokaryotic system is preferable, and a system compatible with a bacterium of the genus Streptomyces is particularly important. The control sequence for use in such a system includes a promoter, a ribosome binding site, a terminator, an enhancer, and the like. A useful promoter is a promoter that functions in a host cell of the genus Streptomyces, and examples thereof include, but are not limited to, pGapdh, pErmE, and pKasO.

A selectable marker may also be included in the plasmid. Various markers are known to be useful in the selection of transformed cell strains and generally include genes whose expression confers a selectable phenotype on the transformed cell when the cell is grown in an appropriate selective medium. Such markers include, for example, genes that confer antibiotic resistance or susceptibility to the plasmid. Alternatively, some polyketides are naturally colored and this feature provides a built-in marker for selecting cells that are successfully transformed with the construct of the present invention.

The plasmid may have a functional sequence that functions in a host cell. Examples of the functional sequence include a plasmid replication origin sequence, a sequence encoding an enzyme for integrating a plasmid into a host genome, and a conjugation origin sequence.

The control sequence, the selectable marker, the functional sequence, and the like can be included in the plasmid by being incorporated into the repeat unit of the DNA construct.

A method for introducing the plasma of the present invention into a suitable host cell is known to those skilled in the art, and typically involves the use of CaCl₂ or divalent cations and other agents such as DMSO. The plasmid may also be introduced into a host cell by electroporation. Once the PKS is expressed, polyketide-producing colonies can be identified and isolated using known techniques. The plasmid of the present invention may be introduced into a host cell using conjugal transfer between bacteria. In one preferred embodiment of the present invention, a region containing DNA that encodes a PKS is transferred from the plasmid of the present invention to a plasmid of E. coli to change a plasmid skeleton (sub-cloning), and then the region is transferred from E. coli to a microorganism of the genus Streptomyces by conjugation to perform introduction into a host cell. The DNA that encodes the PKS is integrated into genome of a host cell such as a microorganism of the genus Streptomyces.

In one embodiment, the plasmid of the present invention may have a replication origin sequence in Bacillus subtilis, E. coli, and a microorganism of the genus Streptomyces, a conjugation origin sequence (OriT) for conjugative transfer of E. coli to a microorganism of the genus Streptomyces, and a sequence encoding integrase required for integration into the genome of a microorganism of the genus Streptomyces. Such a plasmid can be recovered from Bacillus subtilis, transformed into E. coli without sub-cloning for changing the plasmid skeleton, and then transferred from E. coli to a microorganism of the genus Streptomyces by conjugation.

Another preferred embodiment of the present invention is a plasmid containing DNA that encodes a large module PKS. For example, 6-deoxyerythronolide B synthase (DEBS) catalyzes biosynthesis of 6-deoxyerythronolide B which is erythromycin aglycone. Three open reading frames encode DEBS polypeptides and span 32 kb in the ery cluster of the genome of Saccharopolyspora erythraea. This gene is organized in six repeat units each referred to as a "module". Each module encodes a set of active sites, and the active site catalyzes the condensation of additional monomers onto the growth chain during polyketide biosynthesis. Each module may include acyltransferase (AT), β-ketoacyl carrier protein synthase (KS), and an acyl carrier protein (ACP), as well as a subset of reduction active sites (β-ketoreductase (KR), dehydratase (DH), and enoyl reductase (ER)). The number of reduction sites in the module corresponds to a degree of β-keto reduction in each condensation cycle.

The DNA construct can be obtained, for example, by linking a plurality of DNA fragments whose both ends are sticky ends by DNA ligase. Specific examples of the DNA fragment include DNA fragments having ends that can be repeatedly linked to each other while maintaining the order by utilizing complementarity of the base sequence of the sticky end. A structure of the sticky end is not particularly limited as long as it is other than a batch structure (palindrome), including a difference in shapes of the 5' end protrusion and the 3' end protrusion. However, it is preferable that the protruding end can be produced by digestion with a restriction enzyme when the DNA fragment is produced. When an enzyme capable of recognizing a specific sequence and producing a protruding end of an arbitrary sequence in the vicinity thereof is used as the restriction enzyme, the sticky end of the DNA fragment can be made different at each linking site, such that the order of linking is maintained. By appropriately designing the sequence of the sticky end of each DNA fragment, it is possible to obtain a DNA construct in which the respective DNA fragments are arranged in a predetermined order. Examples of these restriction enzymes include, in addition to restriction enzymes used in normal molecular biology, artificial restriction enzymes TALEN and ZNF, and CRISPR technology related enzymes capable of generating a sticky end such as CRISPR-Cpf1, and it is preferable to use Type II restriction enzymes such as AarI, AlwNI, BbsI, BbvI, BcoDI, BfuAI, BglI, BsaI, BsaXI, BsmAI, BsmBI, BsmFI, BspMI, BspQI, BtgZI, DraIII, FokI, PflMI, SfaNI, and SfiI. NEBeta (trademark) Tools can be used to determine optimal sticky ends. The number of bases at the sticky end is preferably 3 to 6, and more preferably 3 or 4. The number of bases contained in one DNA fragment is preferably 1 to 5 kb. The number of bases contained in one DNA fragment may be 2 kb or more, 3 kb or more, or 4 kb or more. Cleavage by one kind of restriction enzyme is preferable for cutting out one DNA fragment for generation of sticky ends. It is not always necessary to obtain all the DNA fragments by digestion with the same kind of restriction enzymes, but the total number of kinds of restriction enzymes to be used is preferably smaller, and is preferably 3 or less, more preferably 2 or less, and still more preferably 1.

The DNA fragment may be prepared, for example, by a method including: a step of preparing a plurality of DNA fragments having sticky ends at both ends by cleaving a plurality of DNA fragment precursors (DNA fragments that produce corresponding DNA fragments when cleaved by a Type II restriction enzyme. For example, it may be a plasmid) with a Type II restriction enzyme; and a step of preparing a DNA construct by linking the plurality of DNA fragments.

The step of preparing the DNA construct by linking the plurality of DNA fragments may be, for example, a step of preparing a DNA construct by linking the plurality of DNA fragments by DNA ligase.

The step of preparing the DNA construct by linking the plurality of DNA fragments may be a step of preparing tandem repeats of DNA that encodes a Type I polyketide synthase (PKS) by mixing solutions each containing the plurality of DNA fragments. The mixing is preferably performed so that a molar concentration ratio of all the DNA fragments is 0.8 to 1.2, preferably 0.9 to 1.1, more preferably 0.95 to 1.05, and still more preferably about 1.0. The DNA fragments may be integrated by mixing the DNA fragments in the presence of an integration plasmid as necessary.

Examples of the DNA ligase include T4 phage and E. coli DNA ligase.

The DNA construct may contain other genes in addition to the DNA that encodes the PKS (for example, DNA that encodes a domain contained in a PKS). For example, when the DNA construct contains a PKS gene, it is preferable to also contain other non-PKS genes in addition to the PKS gene for 1) modification of a final polyketide product, 2) extracellular transport of the polyketide, or 3) imparting resistance to the antibiotic polyketide. In other words, although a portion of the PKS gene is a core biosynthetic portion of the target cluster, the non-PKS gene is also often required.

The number of kinds of DNA fragments is 3 to 60 (kinds), preferably 5 to 50 (kinds), more preferably 8 to 40 (kinds), and still more preferably 10 to 30 (kinds).

The GC content in each DNA fragment may be 65% or less. Preferably, repetition of base sequences of 20 bp or more is prevented from appearing.

For example, when the DNA that encodes the PKS is represented by P-Q-R-S in which four domains are linked in the order of P, Q, R, and S, the DNA construct contains tandem repeats represented by -(P-Q-R-S)ₙ- (n is an integer of 2 or more). It is preferable that a control sequence such as a promoter, a ribosome binding sequence (RBS sequence), or an enhancer that functions in a host cell is added to the 5' end or the 3' end of each ORF (P-Q-R-S) of the DNA construct. In addition, a functional sequence that functions in the host cell and a sequence such as a selectable marker may be added to the 5' end or the 3' end of each ORF (P-Q-R-S) of the DNA construct. In addition, the DNA construct preferably contains a replication origin effective for Bacillus subtilis.

The Bacillus subtilis competent cell is cultured with the DNA construct, such that the DNA construct is incorporated into Bacillus subtilis to form a plasmid containing DNA that encodes a PKS.

A repeat unit containing DNA that encodes a PKS of tandem repeats (including ORF, and as necessary, a control sequence, a functional sequence that functions in a host cell, a selectable marker, and the like) is integrated in the plasmid when the plasmid is formed, such that the tandem repeats of DNA that encodes a PKS are obtained.

It is preferable that a control sequence such as a promoter, a ribosome binding sequence (RBS sequence), or an enhancer that functions in a host cell is added to the plasmid. In addition, the plasmid preferably contains a replication origin effective for Bacillus subtilis. For example, in the case of Bacillus subtilis, it has a θ-type replication mechanism, and specific examples thereof include sequences such as a replication origin contained in plasmids such as pTB19 (Imanaka, T., et al., J. Gen. Microbioi., 130, 1399-1408 (1984)), pLS32 (Tanaka, T and Ogra, M. FEES Lett., 422, 243-246 (1998)), and pAMβ1 (Swinfield, T. J., et al., Gene, 87, 79-90 (1990)).

The terminator is not particularly limited as long as it functions in a host cell, and preferred examples thereof include a terminator derived from fd phage (fd-ter), a terminator derived from T4 phage (T4-ter), and a terminator derived from T7 phage (T7-ter). Among them, a terminator derived from fd phage is particularly preferable in that the stabilization effect described above is greater.

Known ribosome binding sequences (RBS) can be used.

In one embodiment of the present invention, a transformant obtained by introducing a plasmid containing DNA that encodes a PKS into a host cell is cultured, and polyketides can be obtained from the culture. The "culture" means any one of culture supernatant, cultured cells, cultured bacteria, or disrupted cells or bacteria. A method for culturing the transformant of the present invention can be performed according to a method commonly used for culturing a host.

As a medium for culturing the transformant of the present invention, either a natural medium or a synthetic medium may be used as long as the medium contains a carbon source, a nitrogen source, an inorganic salt, and the like that can be assimilated by the host and can efficiently culture the transformant. Examples of the carbon source include carbohydrates such as glucose, galactose, fructose, sucrose, raffinose, and starch, organic acids such as acetic acid and propionic acid, and alcohols such as ethanol and propanol. Examples of the nitrogen source include ammonium salts of inorganic or organic acids such as ammonia, ammonium chloride, ammonium sulfate, ammonium acetate, and ammonium phosphate, and other nitrogen-containing compounds. In addition, peptone, meat extract, corn steep liquor, various amino acids, and the like may be used. Examples of an inorganic substance include monopotassium phosphate, dipotassium phosphate, magnesium phosphate, magnesium sulfate, sodium chloride, iron(II) sulfate, manganese sulfate, copper sulfate, and calcium carbonate.

The culture is usually performed at 28 to 38°C under aerobic conditions such as shaking culture or aeration stirring culture. A pH is adjusted using an inorganic or organic acid, an alkali solution, or the like.

When the culture is performed under the above culture conditions, polyketides can be produced with a high yield.

After the culture, when polyketides are produced in bacteria or cells, the expression product can be collected by subjecting the bacteria or cells to homogenizer treatment or the like to disrupt the bacteria or cells. On the other hand, when polyketides are transported outside bacteria or outside cells, the culture solution is used as it is, or the bacteria or cells are removed by centrifugation or the like. Thereafter, the expression product is collected from the culture by extraction by ammonium sulfate precipitation or the like, and further isolated and purified using various types of chromatography or the like as necessary.

### Examples

Hereinafter, the present invention will be described based on Examples, but the present invention is not limited these Examples.

### Example 1 and Comparative Example 1

### <Design of DNA Fragment>

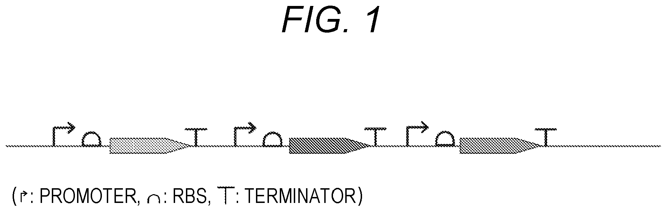

As a target sequence, a sequence in which three DEBS PKS genes were aligned was designed (Fig. 1). Three DEBS PKS gene-encoding sequences were obtained from an erythromycin gene cluster of Saccharopolyspora erythraea. For these three DEBS PKS gene-encoding sequences, codon-optimized DNA was prepared for expression in a host (E. coli) .
GC content before codon optimization (%):
   EryAI:74.2%
   EryAII:74.0%
   EryAIII:73.9%
GC content after codon optimization (%):
   EryAI (SEQ ID NO: 1): 61.7%
   EryAII (SEQ ID NO: 2): 61.5%
   EryAIII (SEQ ID NO: 3): 61.1%

The three DEBS PKS gene-encoding sequences after codon optimization are set forth in SEQ ID NOs: 1 to 3. Control sequences (promoter and RBS sequence) for expression in the host (E. coli) were added before each CDS. A terminator (T) and a spacer sequence were added after each CDS (Fig. 1). BsaI and AarI restriction sites were deleted by changing the synonymous codons.

The target sequence was divided into 28 DNA fragments (each to 1.1 kb), and each fragment was flanked on both sides of the BsaI restriction site. The 28 DNA fragments are set forth in SEQ ID NOs: 4 to 31.

In order to confirm correct assembly, a one-pot Golden Gate reaction was simulated with Genious software.

### <DNA Assembly>

The DNA fragments set forth in SEQ ID NOs: 4 to 31 were ordered from Twist Biosciences and provided as clonal DNA of a standard cloning vector.

### <<Golden Gate Assembly in E. coli (Comparative Example 1)>>:

The 28 DNA fragments were used by being linked in a one-pot Golden-Gate reaction under reaction conditions optimized for a large-scale assembly. An experimental method of the one-pot Golden-Gate referred to ACS Synth. Biol., 2018, 7, 11, 2665-2674.

Specifically, the experimental method was performed according to the following procedure.
1) A concentration of all the DNA fragments was measured with a UV spectrophotometer (Thermofisher Nanodrop) and adjusted to prepare equimolar DNA fragment mixtures. A target vector (pET-24+) was added at a ratio (insert:vector) of 1:1 and 2:1.
2) Assembly was performed using 1.5 µl of BsaI-HFv2 restriction enzyme and 0.5 µl of ligase in a reaction volume of 25 µl.
3) In a thermocycling protocol for a long DNA assembly, the following program was used:
   (5 minutes at 37°C → 5 minutes at 16°C) × 30 cycles, and then 5 minutes at 60°C.
4) 2 µl of the assembly reaction mixture was transformed into NEB 10-beta electrocompetent E. coli cells.
5) Plasmids were extracted from the resulting colonies for validation by restriction patterns.

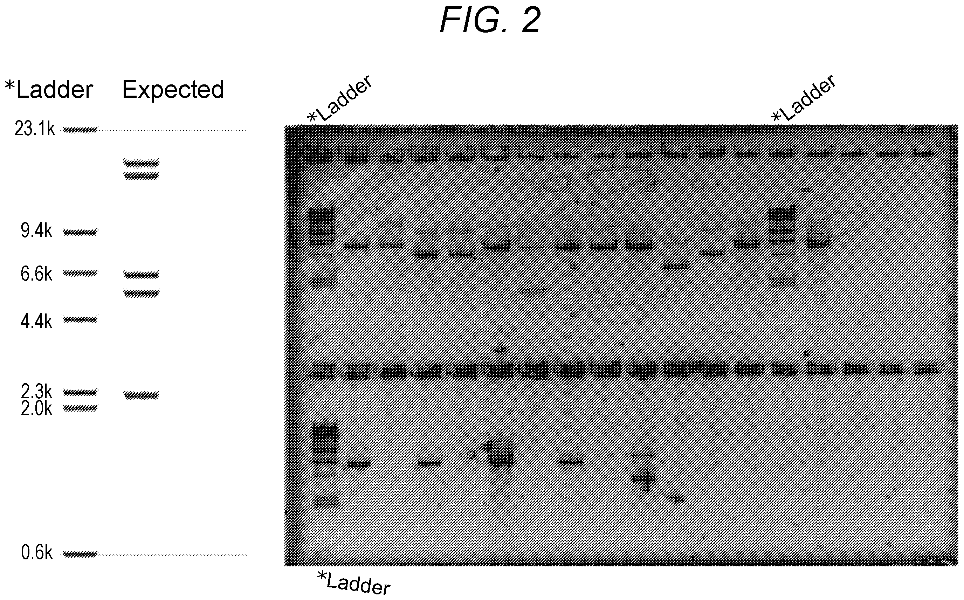

Whether the target sequence was correctly prepared in the extracted plasmid was verified under the following conditions. The results are illustrated in Fig. 2.

### <Experimental Conditions of DEBS PKS Assembly with Golded Gate and E. coli>

100 to 500 ng of the resulting plasmids were dispensed and mixed with 2 ul of 10 × CutSmart buffer, 0.5 ul of BamHI (10 units), and nuclease-free water (up to 20 µl), the mixture was incubated at 37°C for 1 hour, and the target sequence was decomposed into fragments illustrated in Fig. 2.

Electrophoresis was performed at 120 V for 30 minutes in 100 ml of 1 × TAE buffer containing 1 µg/ml ethidium bromide (Sigma), and the gel was stained for 20 minutes. The gel was irradiated with a long wavelength ultraviolet ray (366 mm), and the decomposed DNA fragments were visualized.

### <<Assembly with Bacillus subtilis (Example 1)>>

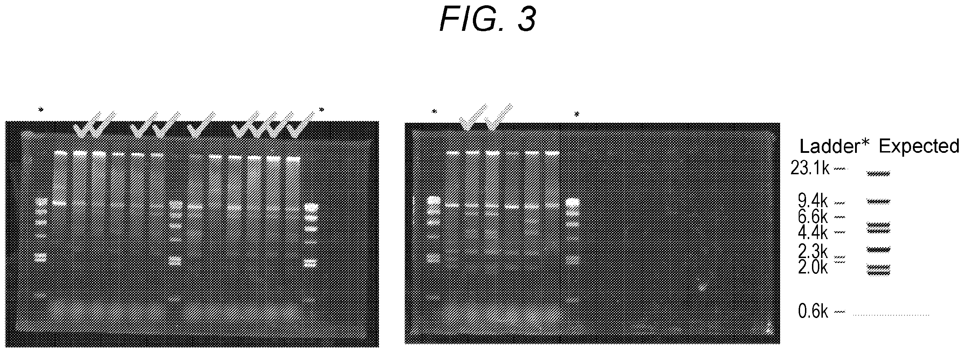

1) All DNA fragments (SEQ ID NOs: 4 to 31) were amplified in E. coli and re-extracted.
2) A concentration of all the DNA fragments (SEQ ID NOs: 4 to 31) was measured with a UV spectrophotometer (Thermofisher Nanodrop) and adjusted to prepare equimolar fragment mixtures.
3) A treatment with DNase (Lucigen Corporation) was performed to remove contaminating linear DNA.
4) The concentration was normalized to 100 ng/ul for each DNA fragment.
5) 500 ng of each DNA fragment was collected in tubes and treated with BsaI-HFv2 restriction enzyme (NEB).
6) In order to purify DNA digested with a restriction enzyme, a phenol chloroform treatment, a butanol treatment, and an ethanol precipitation were performed.
7) Gel extraction using a dialysis tube was performed to remove target fragments from the digested plasmid mixture, and the resulting digested target fragments were purified by ethanol precipitation.
8) Digested fragments were mixed with a digested vector (pET-24 vector), 1 µl of T4 DNA Ligase (TAKARA BIO), a ligation buffer, and the mixture was incubated at 37°C for 3 hours to complete ligation, thereby obtaining a DNA construct containing tandem repeats of DNA that encodes a DEBS PKS.
9) A DNA ligation solution containing the DNA construct was mixed with Bacillus subtilis competent cells, and the cells were spread onto a tetracycline selective plate after a short incubation period at 37°C.
10) After the time for colony growth, a transformant was picked from the plate and grown in 2 ml of LB at 37°C overnight.
11) Plasmid extraction was performed as per the conventional protocol, and whether the resulting DNA was assembled as expected was verified under the same conditions as those in <Experimental Conditions of DEBS PKS Assembly with Golded Gate and E. coli> (Fig. 3).

### <Results>

### Golden Gate Method in E. coli

DEBS assembled with the pET-24 vector showed patterns as illustrated on the left side of Fig. 2 when treated with BamHI, but repeated attempts at assembly in E. coli were not successful over a range of reaction conditions and concentrations (Fig. 2).

### Assembly

When DEBS assembled with the pET-24 vector is treated with BamHI, patterns as illustrated on the right side of Fig. 3 are shown. The green checkmarks indicate such patterns. Assembly of DEBS PKS clusters in Bacillus subtilis was successful and demonstrated with high efficiency (Fig. 3).

## Claims

1. A method for preparing a plasmid containing DNA that encodes a Type I polyketide synthase (PKS), the method comprising a step of introducing a DNA construct containing tandem repeats of DNA that encodes a PKS into a Bacillus subtilis competent cell.

2. The method according to claim 1, further comprising:
a step of preparing a plurality of DNA fragments having sticky ends at both ends by cleaving a plurality of DNA fragment precursors with a Type II restriction enzyme; and
a step of preparing the DNA construct by linking the plurality of DNA fragments.

3. The method according to claim 2, further comprising a step of preparing tandem repeats of DNA that encodes a Type I polyketide synthase (PKS) by mixing solutions each containing the plurality of DNA fragments at a molar concentration ratio of the DNA fragments in each solution of 0.8 to 1.2.

4. The method according to claim 2 or 3, wherein a guanine-cytosine (GC) content in the DNA fragment precursor is 65% or less.

5. The method according to any one of claims 1 to 4, wherein the step of introducing the DNA construct is a step of co-culturing the DNA construct and the Bacillus subtilis competent cell.

6. The method according to any one of claims 1 to 5, further comprising a step of recovering plasmid DNA from Bacillus subtilis into which the DNA construct is introduced.

7. A plasmid comprising DNA that encodes a Type I polyketide synthase (PKS), wherein the plasmid is obtained by the method according to any one of claims 1 to 6.

8. A method for producing a Type I polyketide synthase (PKS), the method comprising: a step of transforming a host cell with a plasmid; and a step of culturing the transformed host cell, wherein the plasmid is the plasmid containing DNA that encodes a PKS obtained by the method according to any one of claims 1 to 6.

9. The method according to claim 8, wherein the host cell is a bacterium of the genus Streptomyces.
